**Europäisches Patentamt**

**European Patent Office**

**Office européen des brevets**

(19)

(11) Numéro de publication: **0 240 385**
**B1**

(12) **FASCICULE DE BREVET EUROPEEN**

(45) Date de publication du fascicule du brevet:
**27.12.90**

(51) Int. Cl.⁵: **A61F 9/02**, H01J 29/18

(21) Numéro de dépôt: **87400419.5**

(22) Date de dépôt: **25.02.87**

(54) **Dispositif de visualisation et de protection d'un faisceau laser, application à des lunettes et à un capot de protection.**

(30) Priorité: **04.03.86 FR 8603035**

(43) Date de publication de la demande:
**07.10.87 Bulletin 87/41**

(45) Mention de la délivrance du brevet:
**27.12.90 Bulletin 90/52**

(84) Etats contractants désignés:
**BE CH DE GB LI NL**

(56) Documents cités:
**WO-A-81/00769**
**US-A- 2 386 855**
**US-A- 4 330 177**
**US-A- 4 336 809**
**US-A- 4 511 225**

(73) Titulaire: **COMMISSARIAT A L'ENERGIE ATOMIQUE**
**Etablissement de Caractère Scientifique Technique et**
**Industriel, 31/33, rue de la Fédération, F-75015 Paris(FR)**

(72) Inventeur: **Le Bihan, André, 11, avenue des**
**Chardonnerets, F-33740 Ares(FR)**

(74) Mandataire: **Mongrédien, André et al, c/o**
**BREVATOME 25, rue de Ponthieu, F-75008 Paris(FR)**

## Description

La présente invention a pour objet un dispositif de visualisation d'un faisceau laser ainsi que de protection contre ce faisceau laser. Le laser peut émettre sur une fréquence fixe, dans le domaine visible ou invisible, ou bien être un laser accordable en fréquence.

L'invention s'applique plus spécialement à des lunettes de protection et à un capot de protection d'une installation optique utilisant une source laser. On rappelle qu'un faisceau laser est un faisceau monochromatique, monodirectionnel, cohérent spatialement et temporellement, éventuellement polarisé.

Les installations comportant des sources laser nécessitant des réglages en présence du faisceau posent le problème de la protection des opérateurs, et en particulier des yeux de ces opérateurs, dès que la puissance de ce faisceau excède 1 mW en régime continu ou impulsionnel lorsque les impulsions sont longues.

En effet, une irradiation laser peut provoquer des brûlures de la peau et des yeux dont les effets sont irréversibles. Dans le cas des yeux, on peut aller jusqu'à la cécité complète.

Le danger dû à un faisceau laser est lié à sa cohérence temporelle et spatiale.

Pour des installations fixes et non destructibles, la protection des opérateurs et notamment de leurs yeux est généralement assurée par un capot réalisé en un matériau opaque au rayonnement laser. Ce capot, entourant totalement les installations, assure une protection complète des opérateurs excepté pendant les périodes de réglage des différents dispositifs desdites installations nécessitant une intervention des opérateurs à l'intérieur du capot.

Pour les installations transitoires ou destructibles ainsi que pour les interventions à l'intérieur d'un capot tel que décrit précédemment, une protection oculaire des opérateurs est assurée par le port de lunettes réalisées en un matériau opaque au rayonnement laser ou présentant un coefficient de transmission connu.

Dans le document US-A 4 511 225, il est décrit des lunettes de protection laser comportant des verres en un matériau absorbant uniformément dans le spectre une partie du faisceau laser. Ces verres sont en particulier des polariseurs.

Les matériaux opaques utilisés en protection laser absorbent une grande partie du spectre de lumière visible empêchant ainsi une vision correcte des dispositifs à régler et de l'environnement.

En outre, l'emploi de ces matériaux dans les capots et les lunettes de protection occultant totalement le rayonnement laser, ne permet pas à l'opérateur de savoir où se trouve le rayonnement occulté et en particulier de détecter un faisceau parasite frappant les parois internes du capot ou les lunettes de protection. Or, l'ouverture du capot ou le retrait des lunettes de protection présente un sérieux danger.

Dans le document US-A 4 330 177, il est décrit des rideaux ou écrans servant à protéger le personnel de surveillance et les passants du rayonnement émis par un arc à souder. Ces rideaux ou écrans se présentent sous forme monobloc. Ils assurent une discrimination spatiale du spectre de lumière au moyen de colorants, une discrimination spatiale en longueur d'onde par réfraction superficielle, une diminution de l'intensité lumineuse par dispersion et un décalage en longueurs d'onde par fluorescence.

Les rideaux ou écrans sont utilisés pour une protection légère contre un rayonnement polychromatique, multidirectionnel, non polarisé et non cohérent.

Compte tenu de la faible performance exigée pour ces rideaux ou écrans, de leur complexité et de leur utilisation en lumière incohérente, l'homme du métier, dans la protection laser, n'est pas conduit à utiliser tout ou partie des éléments constituant ces rideaux ou écrans.

Par ailleurs, il est décrit dans le brevet US-A 4 320 940 des lunettes de soleil filtrant certaines radiations du soleil et ayant pour but d'empêcher l'émission par ces filtres d'une lumière visible nuisant à l'esthétique.

A cet effet, les verres de ces lunettes sont recouverts de plusieurs couches minces de matériaux fluorescents particuliers.

Ces matériaux sont utilisés pour le confort des yeux et l'esthétique et en présence d'une lumière non cohérente. L'homme du métier n'est donc pas enclin à utiliser ces matériaux pour une protection efficace contre les rayonnements. lasers, cohérents.

Dans ces deux documents, la lumière à filtrer est beaucoup moins dangereuse que celle d'un faisceau laser. En outre, le "danger", c'est-à-dire la source de lumière, est parfaitement identifié.

Dans une installation optique utilisant une source laser, le danger n'est pas toujours visible. En effet, des rayonnements lasers parasites, provenant d'une réflexion sur un élément optique (lentille, miroir), ne sont perceptiles à l'oeil nu que s'ils rencontrent une paroi diffusante ou réfléchissante. Or, même dans ce cas particulier, d'une perception d'un rayonnement laser parasite, la direction et la provenance de ce rayonnement ne sont pas connues.

La présente invention a justement pour but un dispositif de visualisation et de protection d'un faisceau laser cohérent et une application à des lunettes et à un capot de protection, permettant de remédier aux différents inconvénients ci-dessus.

En particulier, le dispositif de protection de l'invention permet non seulement d'assurer une protection efficace du personnel, comme les dispositifs de l'art antérieur, mais aussi de renseigner ce dernier sur la présence éventuelle d'un faisceau parasite dangereux et de permettre un réglage en toute sécurité d'installations optiques complexes.

De façon plus précise, l'invention a pour objet un dispositif de protection d'un faisceau laser cohérent, visible ou non, ayant une longueur d'onde donnée permettant aussi la visualisation de ce faisceau laser.

Selon l'invention, le dispositif de protection comprend une couche d'un matériau, interceptant le fai- ceau laser et assurant l'absorption d'une partie du faisceau laser, ce matériau étant en outre partiellement transparent à une partie du spectre de la lumière visible et se caractérise en ce que le matériau assure, de plus, la transformation par fluorescence de l'autre partie du faisceau laser en un faisceau de lumière incohérente visible afin de visualiser le faisceau laser.

Dans ce dispositif, seule la partie du faisceau laser réfléchie à la surface du matériau est encore dangereuse. Toutefois, cette partie réfléchie est infime.

La transformation d'un faisceau (laser) de lumière cohérente, visible ou non, en un faisceau de lumière incohérente visible permet de visualiser le faisceau incident et donc d'identifier le danger. En particulier, elle permet de prévenir l'opérateur de la présence d'un faisceau parasite éventuellement dangereux à l'intérieur d'un capot de protection par visualisation directe de l'impact du faisceau sur ledit capot, soit au contact des lunettes de protection par illumination de celles-ci.

Par ailleurs, cette transformation permet d'éliminer tous les dangers (tels que brûlures) pour les opérateurs et notamment pour leurs yeux, liés à la cohérence du faisceau incident.

La visualisation et la destruction de la cohérence du faisceau laser frappant le dispositif de l'invention permettent d'assurer une protection parfaite, des opérateurs.

De façon avantageuse, le matériau entrant dans la constitution du dispositif de protection de l'invention peut être disposé sur un substrat transparent.

Selon une variante de réalisation du dispositif de protection ci-dessus, le matériau est formé d'une substance, ayant des propriétés de fluorescence et des propriétés d'absorption du faisceau incident, noyée dans un liant notamment solide.

Selon une autre variante de réalisation du dispositif de protection ci-dessus, le matériau est formé d'un mélange de deux matériaux, l'un ayant des propriétés de fluorescence, l'autre des propriétés d'absorption du faisceau incident.

Selon un second mode de réalisation, le dispositif selon l'invention comprend une couche d'un premier matériau, assurant la transformation par fluorescence d'au moins une première partie du faisceau laser en un faisceau de lumière incohérente visible, et une couche d'un second matériau assurant l'absorption d'au moins une seconde partie du faisceau laser et étant en outre partiellement transparent à une partie du spectre de la lumière visible, les deux couches étant disposées l'une au-dessus de l'autre, la couche de premier matériau étant destinée à être disposée du côté du faisceau laser.

Le matériau fluorescent peut éventuellement absorber une faible partie du faisceau laser; ceci justifie l'utilisation des expressions «transformation d'au moins une première partie» et «absorption d'au moins une seconde partie». Le principal est qu'une partie du faisceau laser incident soit absorbée et que l'autre partie soit transformée en lumière visible.

Dans ce mode de réalisation, le dispositif de protection peut avantageusement comprendre un substrat transparent recouvert sur une première face de la couche de premier matériau et sur une deuxième face, opposée à la première, de la couche de second matériau.

L'invention a aussi pour objet des lunettes de visualisation et de protection d'un faisceau laser comportant des verres constitués par le dispositif décrit ci-dessus.

Par «verres» il faut comprendre les éléments des lunettes situés en face des yeux, bien que ces éléments ne soient pas spécialement en verre.

L'invention a encore pour objet un capot de visualistion et de protection d'un faisceau laser, caractérisé en ce qu'il présente en section la forme d'un U dont l'une des branches est articulée et en ce qu'il comprend une partie rigide, constituée du dispositif de protection décrit ci-dessus, et une partie souple en matériau opaque au rayonnement laser, solidaire de la partie rigide, la partie souple formant l'extrémité de la branche articulée.

D'autres caractéristiques et avantages de l'invention ressortiront mieux de la description qui va suivre, donnée à titre illustratif et non limitatif, en référence aux figures annexées, dans lesquelles:

La fig. 1a représente schématiquement une installation optique logée dans un capot de protection selon l'invention;

la fig. 1b représente schématiquement, en perspective, un capot de protection selon l'invention, selon un premier mode de réalisation;

la fig. 2 représente schématiquement une variante du dispositif de la fig. 1b;

la fig. 3 représente schématiquement une partie du dispositif de protection de l'invention, selon un deuxième mode de réalisation;

la fig. 4 représente schématiquement une variante du dispositif de la fig. 3, et

la fig. 5 représente schématiquement des lunettes de protection conformément à l'invention.

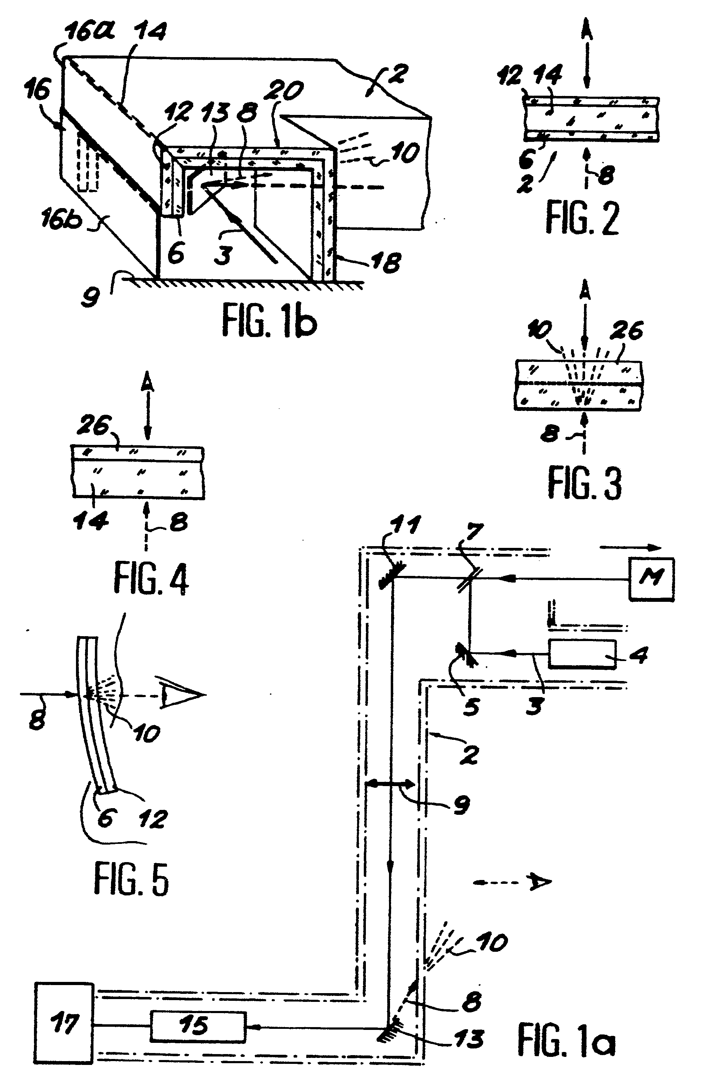

Sur la fig. 1a, on a représenté une installation optique 1 servant à mesure par effet Doppler, la vitesse de déplacement d'un mobile M. Cette installation 1 entourée (fig. 2) sur trois côtés par un capot 2 de protection comprend une source de lumière cohérente 4 ou source laser émettant un faisceau 3. Cette source laser 4 peut émettre dans le visible ou dans l'invisible et en particulier dans l'ultraviolet, selon une longueur d'onde fixe ou bien accordable.

L'installation optique 1 comprend un miroir 5 suivi d'une lame séparatrice 7 servant à envoyer le faisceau laser 3 sur le mobile M. Le faisceau lumineux réfléchi par le mobile est focalisé grâce à une lentille 9 et envoyé, via des miroirs 11 et 13, sur un interféromètre de Pérot-Fabry 15.

L'interféromètre 15 fournit un anneau dont le diamètre est directement lié à la longueur d'onde du signal lumineux reçu par l'interféromètre.

En comparant le diamètre des anneaux obtenus, après réflexion du faisceau laser sur le mobile M au repos, puis sur le mobile en déplacement, on peut déduire la vitesse de déplacement de ce mobile.

Une caméra ultra-rapide 17 permet d'enregistrer à différents moments la vitesse d'un même mobile M.

Le capot 2 permet d'assurer la protection d'un observateur, notamment contre les faisceaux laser parasites 8 provenant des éléments optiques de l'installation 1.

Sur la figure 1b, on a représenté, en perspective une partie du capot 2 assurant une protection contre un faisceau parasite 8 provenant du miroir 13. Ce capot présente en section la forme d'un U comportant deux branches 16 et 18 parallèles et une base 20. La protection contre le rayonnement parasite 8 sur le quatrième côté est ici assurée par la table 9 supportant l'installation.

Bien entendu, le capot 2 peut être conçu pour entourer totalement la source laser, ainsi que l'optique associée.

La branche 16 du U est montée sur la base du U par l'intermédiaire d'une charnière 14. Ceci permet d'escamoter la branche 16 et donc d'ouvrir le capot 2, lorsque l'on désire effectuer un réglage de l'installation optique. Dans ce cas, l'opérateur doit être pourvu de lunettes de protection (figure 5) conformes à l'invention.

La partie 16a de la branche 16 montée à articulation, la base 20 et la branche 18 sont rigides, tandis que l'extrémité 16b de la branche 16 est souple.

Cette extrémité 16b est réalisée en un matériau opaque au rayonnement laser émis par la source 4 et en particulier en un tissu noir ignifugé. Cette extrémité souple 16b permet à l'observateur d'introduire les mains dans le capot 2 et d'effectuer un réglage de l'installation, sans avoir à ouvrir le capot 2. Dans ce cas, l'opérateur peut ne pas porter de lunettes de protection selon l'invention.

Les parties rigides 16a, 18 et 20 du capot sont formées d'une couche 6 d'un premier matériau servant à transformer une partie du faisceau parasite de lumière cohérente 8, faisceau qui est dangereux pour l'oeil au-dessus d'une certaine puissance lumineuse, en des faisceaux de lumière incohérente visible 10 sans danger pour l'oeil. Cette couche de matériau 6 est apte à délivrer sous l'impact du rayonnement laser 8, une luminescence, schématisée par les faisceaux 10, présentant une longueur d'onde visible différente de celle du rayonnement laser incident 8.

Les parties rigides 16a, 18 et 20 du capot 2 comprennent de plus une couche 12 d'un second matériau recouvrant entièrement la couche de matériau 6. Cette couche de matériau 12 assure l'absorption ou le filtrage de l'énergie lumineuse, véhiculée par le rayonnement laser incident 8, n'ayant pas été transformée en énergie lumineuse visible incohérente par la couche 6.

Cette couche de matériau 12, opaque au rayonnement incident 8, doit être partiellement transparente à une partie du spectre de la lumière visible, notamment pour permettre une vision correcte de l'environnement du capot 2.

Sur la figure 1a, la couche de matériau 6 est disposée du côté du rayonnement laser 8 et la couche de matériau 12 est disposée du côté de l'observateur dont on n'a représenté que l'oeil. Ceci permet d'assurer à coup sûr une protection efficace, puisque la partie du rayonnement laser 8 non transformée en lumière incohérente visible est automatiquement interceptée par la couche de matériau 12.

Dans cette disposition, il est nécessaire que la couche de matériau 6 délivre, sous l'impact des faisceaux laser, une luminescence ayant une longueur d'onde pouvant être transmise sans ou peu d'atténuation par la couche de matériau 12.

Lorsque le capot 2 est fermé, le faisceau laser 3, issu de la source 4, orienté parallèlement aux parois 16, 18 et 20 du capot 2, n'est pas visible. L'alignement optique des éléments 5, 7, 11, 9, 13, 15 de l'installation peut alors être réalisé en introduisant par la partie souple 16b du capot un écran fluorescent, par exemple en Dacryl-fluo-orange, permettant, par interception du faisceau laser, la visualisation de ce dernier.

Un capot bicouche 2, tel que représenté sur la figure 1b, a permis de protéger efficacement le personnel contre un rayonnement laser 8 à argon ionisé de couleur verte, la longueur d'onde étant voisine de 514,5 nm, véhiculant une énergie continue de l'ordre de 6W, tout en visualisant le trajet dudit rayonnement.

Les parties rigides du capot étaient constituées d'une couche 6 de 3 mm d'épaisseur réalisée en un matériau commercialisé sous le nom de Dacryl-fluo-orange (Altuglas® dopé), fabriqué par la Société Dacryl (France), émettant, sous l'effet d'un rayonnement incident, une fluorescence orange et d'une couche 12 de 3 mm, dirigée du côté de l'observateur, réalisée en un matériau commercialié sous le nom de Altuglas® rèf.500 orange, fabriqué par la Société EIC (France), assurant l'absorption du faisceau lumineux incident et résiduel, c'est-à-dire non transformé en lumière incohérente visible.

De tels matériaux présentent l'avantage de pouvoir être réalisés sous n'importe quelle forme adaptée aux besoins de la protection. En outre, ils peuvent être utilisés pour la visualisation et la protection d'un faisceau laser dont la longueur d'onde va de 488 nm à 580 nm.

Sur la figure 2, on a représenté une variante du dipositif de protection représenté sur la figure 1b. Dans cette variante, les deux couches de matériau 6 et 12 au lieu d'être disposées au contact l'une de l'autre sont séparées par un substrat transparent 14, tel que du Plexiglas®. L'interposition d'un substrat 14 permet de diminuer l'épaisseur des couches des matériaux 6 et 12 tout en assurant une bonne rigidité du dispositif de protection 2 ou capot.

La couche de matériau 12, dirigée du côté de l'observateur, se présente sous la forme d'un film absorbant la lumière en résine époxy teintée orange que l'on colle sur le substrat et la couche de matériau 6, dirigée du côté du faisceau de lumière cohérente incident 8, peut se présenter sous la forme d'une peinture fluorescente (vernis à ongles par exemple) ou d'une couche de résine époxy telle que l'Araldite® renfermant 1% en poids de fluorescéine. Cette réalisation particulière du dispositif de l'invention est utilisable pour une protection contre un faisceau laser de 514,5 nm.

Sur la figure 3, on a représenté un autre mode de réalisation du dispositif de protection conformément à l'invention. Dans ce mode de réalisation, le matériau constituant le dispositif de protection, dispositif qui constitue une partie d'un capot de protection (figures 1a, 1b) ou un verre de lunette (figure 5), permettant de transformer par fluorescence une partie d'un faisceau 8 de lumière cohérente visible ou non en un faisceau de lumière incohérente visible 10 et d'absorber la partie du faisceau incident non transformée, peut être réalisé en une seule couche de matériau 26.

Cette unique couche 26 de matériau peut être constituée d'une substance ayant à la fois les propriétés de fluorescence et d'absorption du faisceau laser incident, noyée dans un liant notamment à l'état solide.

Un matériau monocouche 26 composé de fluorescéine noyée dans une colle époxy telle que l'Araldite® , a permis, sous l'impact d'un faisceau laser de couleur verte ayant une longueur d'onde voisine de 514,5 nm et une énergie de 5 Watts continus, de convertir une partie de l'énergie lumineuse de ce faisceau incident en une lumière non cohérente visible par émission d'une fluorescence dans le rose orangé.

Ce matériau monocouche permet aussi d'assurer, lorsque la quantité de fluorescéine contenue dans le liant est suffisante, c'est-à-dire de 1 à 10% en poids, la fonction du filtrage ou d'absorption du faisceau lumineux incident résiduel, c'est-à-dire non transformé en faisceau lumineux incohérent visible. Plus la concentration en fluorescéine est élevée, plus le matériau absorbe le rayonnement laser.

Un tel matériau solide présente l'avantage d'être réalisé de façon aisée et d'être facilement employé pour réaliser, par moulage, des dispositifs de protection ayant des formes complexes adaptées aux types de protection souhaités. En outre, ce matériau est réversible, c'est-à-dire que les deux surfaces opposées du matériau peuvent être orientées indifféremment vers le rayonnement laser ou l'opérateur.

Cependant, le liant en Araldite® présente une certaine fragilité aux fortes énergies du rayonnement de laser incident (supérieures à 5 W).

Pour remédier à cet inconvénient, il est possible de disposer le matériau monocouche 26, à l'état solide, comme représenté sur la figure 4, sur un substrat transparent 14, par exemple en Plexiglas®.

Lorsque le matériau assurant la transformation en lumière incohérente visible d'un faisceau de lumière cohérente et son absorption, tel qu'un faisceau laser, est constitué en une seule couche de matériau 26 solide, celui-ci peut aussi être formé d'un mélange de deux matériaux, l'un ayant des propriétés de transformation du faisceau de lumière cohérente, visible ou non, en un faisceau de lumière incohérente visible, c'est-à-dire des propriétés de fluorescence, l'autre matériau présentant des propriétés d'absorption du faisceau incident de lumière cohérente.

Un tel matériau monocouche 26 peut être constitué par du Dacryl fluo-rouge fabriqué par la Société Dacryl (France). Ce matériau a permis d'assurer une protection efficace contre un faisceau laser de couleur verte, de longueur d'onde voisine de 514,5 nm. Ce matériau monocouche permet de réaliser des dispositifs de protection présentant n'importe quelle forme. Par ailleurs, ce matériau présente des propriétés de réversibilité (pas d'orientation particulière vis-à-vis du rayonnement incident).

Comme précédemment, ce matériau monocouche solide formé d'un mélange de deux matériaux peut être utilisé seul, en couches épaisses (figure 3) ou bien être réalisé sous la forme d'une couche mince disposée sur un substrat transparent (14) (figure 4).

Les dispositifs de protection selon l'invention sont en particulier des capots entourant tout ou partie d'une installation optique utilisant un faisceau de lumière cohérente ou faisceau laser (figures 1a, 1b), de simples écrans ou bien des verres de lunettes de protection, comme représenté sur la figure 5.

Ces verres de protection peuvent être réalisés sous forme d'un matériau monocouche ou bicouche ayant des propriétés de fluorescence et d'absorption d'un rayonnement laser, tel que ceux décrits précédemment.

Bien entendu, dans le cas d'un matériau bicouche, tel que représenté sur la figure 5, la couche de matériau 6 ayant les propriétés de fluorescence, c'est-à-dire permettant de transformer le faisceau de lumière cohérente, visible ou non, en un faisceau de lumière incohérente visible, doit être disposée du côté du faisceau incident 8 et la couche de matériau 12, ayant les propriétés d'absorption du faisceau de lumière cohérente incident, doit être disposée du côté de l'oeil de l'observateur ; cette couche de matériau 12 absorbe la partie du faisceau incident non transformée en lumière incohérente visible par la couche de matériau 6.

Les différents matériaux constituant le dispositif de protection de l'invention décrit prédédemment n'ont bien entendu été donnés qu'à titre d'exemples. D'autres matériaux ayant des propriétés de fluorescence et/ou d'absorption d'un faisceau de lumière cohérente, visible ou non, peuvent être utilisés, à condition toutefois d'être transparents à une bonne partie du spectre de lumière visible de façon à permettre une vision correcte ; cette dernière condition est particulièrement souhaitée pour des lunettes de protection.

Par ailleurs, ces exemples ont été décrits dans le cas d'une protection contre un rayonnement laser émettant dans le vert, mais bien entendu, l'invention est applicable de façon générale à toute lumière cohérente, visible ou non, qui respecte la loi de Stokes. Toutefois, les matériaux absorbants et/ou fluorescents utilisables doivent être spécifiques d'une longueur d'onde ou d'une gamme limitée de longueurs d'onde afin de laisser passer la majeure partie du spectre de lumière visible pour permettre une vision correcte des installations à régler et de l'environnement.

De même, les différents exemples ont été donnés pour un laser émettant sur une fréquence fixe, mais bien entendu, l'invention s'applique aussi à des lasers accordables en longueur d'onde à condition toutefois que le spectre balayé par le laser ne comprenne pas tout le spectre visible, ce qui ne permettrait pas une vision correcte en particulier des dispositifs à régler et de l'environnement des installations utilisant de tels lasers, et que le matériau utilisé présente la propriété d'absorber le rayonnement laser, dans toute la plage d'accord, et la propriété d'émettre, pout tout ce spectre d'accord, une luminescence visible.

Pour un laser accordable en longueur d'onde, le dispositif de protection selon l'invention peut être constitué de plusieurs couches de matériau empilées et/ou de substances différentes noyées dans un liant, chacune de ces couches et/ou de ces substances émettant une fluorescence pour une longueur d'onde donnée du faisceau incident cohérent et/ou absorbant l'énergie dudit faisceau pour une longueur d'onde donnée.

## Revendications

1. Dispositif de protection d'un faisceau laser cohérent ayant une longueur d'onde, comprenant une couche d'un matériau (26) interceptant le faisceau laser et assurant l'absorption d'une partie de ce faisceau laser (8), ce matériau (26) étant en outre partiellement transparent à une partie du spectre de la lumière visible, caractérisé en ce que le matériau assure, de plus, le transformation par fluorescence de l'autre partie du faisceau laser en un faisceau de lumière incohérente visible (10), afin de visualiser le faisceau laser (8).

2. Dispositif selon la revendication 1, caractérisé en ce qu'il comprend un substrat transparent (14) sur lequel est disposé ledit matériau (26).

3. Dispositif selon la revendication 1, caractérisé en ce que le matériau (26) est formé d'une substance ayant des propriétés de fluorescence et des propriétés d'absorption du faisceau incident (8), noyée dans un liant.

4. Dispositif selon la revendication 3, caractérisé en ce que le liant est un solide.

5. Dispositif selon la revendication 1, caractérisé en ce que le matériau (26) est formé d'un mélange de deux matériaux, l'un ayant des propriétés de fluorescence, l'autre des propriétés d'absorption du faisceau laser (8).

6. Dispositif de visualisation et de protection d'un faisceau laser cohérent ayant une longueur d'onde donnée, comprenant une couche d'un premier matériau (6), interceptant le faisceau laser et assurant la transformation par fluorescence d'au moins une première partie du faisceau laser (8) en un faisceau de lumière incohérente visible (10), et une couche d'un second matériau (12) assurant l'absorption d'au moins une seconde partie du faisceau laser (8) et étant en outre partiellement transparent à une partie du spectre de la lumière visible, les deux couches (12, 6) étant disposées l'une au-dessus de l'autre, la couche de premier matériau étant destinée à être disposée du côté du faisceau laser (8).

7. Dispositif selon la revendication 6, caractérisé en ce qu'il comprend un substrat transparent (14) recouvert sur une première face de la couche de premier matériau (12) et recouvert sur une deuxième face, opposée à la première, de la couche de second matériau (6).

8. Lunettes de visualisation et de protection d'un faisceau laser ayant une longueur d'onde donnée, comportant des verres, caractérisées en ce que les verres sont constitués par le dispositif selon l'une quelconque des revendications 1 à 7.

9. Capot de visualisation et de protection d'un faisceau laser cohérent ayant une longueur d'onde donnée, caractérisé en ce qu'il présente en section la forme d'un U dont l'une des branches (16) est articulée et en ce qu'il comprend une partie rigide (16a, 18, 20), constituée du dispositif selon l'une quelconque des revendications 1 à 7, et une partie souple (16b) en matériau opaque au rayonnement laser, solidaire de la partie rigide, la partie souple (16b) formant l'extrémité de la branche articulée (16).

10. Capot de protection selon la revendication 9, caracérisé en ce que la partie souple (16b) est réalisée en tissu noir ignifugé.

## Patentansprüche

1. Vorrichtung zum Schutz vor einem kohärenten Laserlichtbündel mit gegebener Wellenlänge mit einer Schicht aus einem Material (26), das das Laserlichtbündel unterbricht und die Absorption eines Teiles dieses Laserlichtbündels (8) sicherstellt, wobei dieses Material (26) außerdem teilweise transparent für einen Teil des sichtbaren Spektrums ist, dadurch gekennzeichnet, daß das Material außerdem die Umwandlung des anderen Teils des Laserlichtbündels durch Fluoreszenz in ein inkohärentes, sichtbares Lichtbündel (10) sicherstellt, um das Laserlichtbündel (8) sichtbar zu machen.

2. Vorrichtung nach Anspruch 1, dadurch gekennzeichnet, daß sie ein transparentes Substrat (14) umfaßt, auf dem das Material (26) abgeschieden ist.

3. Vorrichtung nach Anspruch 1, dadurch gekennzeichnet, daß das Material (26) aus einer Substanz besteht, die Fluoreszenzeigenschaften und Absorptionseigenschaften für das einfallende Lichtbündel (8) besitzt, die in einem Binder eingebettet ist.

4. Vorrichtung nach Anspruch 3, dadurch gekennzeichnet, daß der Binder ein Festkörper ist.

5. Vorrichtung nach Anspruch 1, dadurch gekennzeichnet, daß das Material (26) aus einer Mischung zweier Materialien besteht, wovon eines fluoreszierend ist und das andere Absorptionseigenschaften für das Laserlichtbündel (8) hat.

6. Vorrichtung zum Sichtbarmachen von und zum Schutz vor einem kohärenten Laserlichtbündel mit gegebener Wellenlänge mit einer Schicht aus einem ersten Material (6), das das Laserlichtbündel unterbricht und die Umwandlung wenigstens eines ersten Teils des Laserlichtbündels (8) in ein Bündel inkohärenten, sichtbaren Lichts (10) durch Fluoreszenz sicherstellt, und einer Schicht aus einem zweiten Material, das die Absorption wenigstens eines zweiten Teils des Laserlichtbündels (8) sicherstellt und außerdem teilweise für einen Teil des Spektrums des sichtbaren Lichts transparent ist, wobei die beiden Schichten (12, 6) übereinander angeordnet sind und die Schicht aus dem ersten Material dazu bestimmt ist, auf der Seite des Laserlichtbündels (8) angeordnet zu werden.

7. Vorrichtung nach Anspruch 6, dadurch gekennzeichnet, daß sie ein transparentes Substrat (14) umfaßt, das auf einer ersten Seite mit der Schicht aus dem ersten Material (12) und auf einer zweiten, der ersten gegenüberliegenden Seite mit der Schicht aus dem zweiten Material (6) bedeckt ist.

8. Brille zum Sichtbarmachen von und zum Schutz vor einem kohärenten Laserlichtbündel mit gegebener Wellenlänge mit Gläsern, dadurch gekennzeichnet, daß die Gläser aus Vorrichtungen nach einem der Ansprüche 1 bis 7 bestehen.

9. Abdeckhaube zum Sichtbarmachen von und zum Schutz vor einem kohärenten Laserlichtbündel mit gegebener Wellenlänge, dadurch gekennzeichnet, daß sie im Querschnitt eine U-Form aufweist, wovon ein Zweig (16) gelenkig befestigt ist, und daß sie einen steifen Teil (16a, 18, 20), der aus der Vorrichtung nach einem der Ansprüche 1 bis 7 besteht, und einen flexiblen Teil (16b) besitzt, der aus einem für Laserlicht undurchsichtigen Material besteht und an dem steifen Teil befestigt ist, wobei der flexible Teil (16b) das Ende des gelenkig befestigten Zweigs (16) bildet.

10. Schutzabdeckhaube nach Anspruch 9, dadurch gekennzeichnet, daß der flexible Teil (16) aus einem schwarzen, feuersicheren Gewebe besteht.


**Claims**

1. Device for protection from a coherent laser beam having a given wavelength, comprising a layer of a material (26) intercepting the laser beam and ensuring the absorption of a portion of this laser beam (8), this material (26) also being partially transparent to a portion of the spectrum of visible light, characterized in that the material also ensures the transformation by fluorescence of the other portion of the laser beam into a beam (10) of visible incoherent light, to see the laser beam (8).

2. Device according to claim 1, characterized in that it comprises a transparent substrate (14) on which said material (26) is placed.

3. Device according to claim 1, characterized in that the material (26) is made of a substance having properties of fluorescence and properties for absorption of the incident beam (8), embedded in a binder.

4. Device according to claim 3, characterized in that the binder is a solid.

5. Device according to claim 1, characterized in that the material (26) is made of a mixture of two materials, one having properties of fluorescence, the other properties for absorption of the laser beam (8).

6. Device for visualizing and protecting from a coherent laser beam having a given wavelength, comprising a layer of a first material (6), intercepting the laser beam and assuring the transformation by fluorescence of at least a first portion of the laser beam (8) into a beam (10) of visible incoherent light, and a layer of a second material (12) ensuring the absorption of at least a second portion of the laser beam (8) and, in addition, being partially transparent to a portion of the spectrum of visible light, the two layers (12, 6) being placed one above the other, the layer of first material being intended to be placed on the side of the laser beam (8).

7. Device according to claim 6, characterized in that it comprises a transparent substrate (14) covered on a first face with the layer of first material (12) and covered on a second face, opposite the first, with the layer of second material (6).

8. Glasser for display of and protection from a laser beam having a given wavelength, comprising lenses, characterized in that the lenses consist of the device according to any one of the claims 1 to 7.

9. Hood for the visualization of and protection from a coherent laser beam having a given wavelength, characterized in that it has in section the shape of a U, one of whose legs (16) is hinged and in that it comprises a rigid part (16a, 18, 20), consisting of the device according to any one of the claims 1 to 7 and a flexible part (16b) of light-absorbing material, integral with the rigid part, the flexible part (16b) forming the end of the hinged leg (16).

10. Protective hood according to claim 9, characterized in that the flexible part (16b) is made of fireproofed black fabric.

EP 0 240 385 B1

FIG. 1b

FIG. 2

FIG. 3

FIG. 4

FIG. 5

FIG. 1a